# EUROPEAN PATENT SPECIFICATION

(11) **EP 1 403 284 B1**
(45) Date of publication and mention of the grant of the patent: **10.12.2008**
(21) Application number: 02734987.7
(22) Date of filing: 27.05.2002
(51) Int. Cl.: C07K 14/705, C07K 7/08, A61K 38/10

(54) **HAb18G/CD147, ITS ANTAGONIST AND APPLICATION**
HAb18G/CD147, DESSEN ANTAGONIST UND ANWENDUNG
HAb18G/CD147, SON ANTAGONISTE ET SON UTILISATION

(30) Priority: 25.05.2001 CN 01115274; 28.09.2001 CN 01131735
(43) Date of publication of application: 31.03.2004
(73) Proprietor: Chen, Zhinan, Shanxi 710032 (CN)
(72) Inventor: CHEN, Zhinan, Nat.Base Cell Engin.of 863 Project, Shanxi 710032 (CN); SHANG, Peng, Nat.Base Cell Engin.of 863 Project, Shanxi 710032 (CN); LI, Yu, Nat.Base Cell Engin.of 863 Project, Shanxi 71032 (CN); QIAN, Airong, Nat.Base Cell Engin.of Project 863, Shanxi 710032 (CN); ZHU, Ping, Nat.Base Cell Engin.of 863 Project, Shanxi 710032 (CN); XING, Jinliang, Nat.Base Cell Engin.of 863 Project, Shanxi 710032 (CN)
(74) Representative: Vossius & Partner
(86) International application number: PCT/CN2002/000356
(87) International publication number: WO 2002/094875

(56) References cited:
- WO-A2-99/45031
- JIANG J L ET AL: "The involvement of HAb18G in regulating store-operated calcium entry in human hepatoma cells" FASEB JOURNAL, vol. 15, no. 5, 8 March 2001 (2001-03-08), page A1115, XP008038955 & ANNUAL MEETING OF THE FEDERATION OF AMERICAN SOCIETIES FOR EXPERIMENTAL BIOLOGY ON EXPERIMENTAL BIOL; ORLANDO, FLORIDA, USA; MARCH 31-APRIL 04, 2001 ISSN: 0892-6638
- JIANG J L ET AL: "The involvement of HAb18G/CD147 in regulation of store-operated calcium entry and metastasis of human hepatoma cells." THE JOURNAL OF BIOLOGICAL CHEMISTRY. 14 DEC 2001, vol. 276, no. 50, 14 December 2001 (2001-12-14), pages 46870-46877, XP002339199 ISSN: 0021-9258 & CHEN Z.N. ET AL: "Structure and function analysis of hepatoma associated factor HAb18G" CHINESE JOURNAL OF CELLULAR AND MOLECULAR IMMUNOLOGY, vol. 15, 1999, page 34, ISSN: 1007-8738
- DEROOCK ET AL: "Synthetic peptides inhibit adhesion of human tumor cells to extracellular matrix proteins" CANCER RESEARCH, AMERICAN ASSOCIATION FOR CANCER RESEARCH, BALTIMORE, MD, US, vol. 61, no. 8, 15 April 2001 (2001-04-15), pages 3308-3313, XP002184132 ISSN: 0008-5472
- LI Y ET AL: "Inhibitory effects of antisense RNA of HAb18G/CD147 on invasion of hepatocellular carcinoma cells in vitro" WORLD JOURNAL OF GASTROENTEROLOGY 2003 CHINA, vol. 9, no. 10, 2003, pages 2174-2177, XP002349398 ISSN: 1007-9327
- QIAN A-R ET AL: "Effects of HAb18G/CD147 antagonistic peptides on invasion and metastasis of HCC" WORLD CHINESE JOURNAL OF DIGESTOLOGY 01 MAR 2003 CHINA, vol. 11, no. 3, 1 March 2003 (2003-03-01), pages 255-259, XP002349399 ISSN: 1009-3079
- BISWAS C. ET AL.: 'THE HUMAN TUMOR CELL-DERIVED COLLAGENASE STIMULATORY FACTOR (RENAMED EMMPRIN) IS A MEMBER OF THE IMMUNOGLOBULIN SUPERFAMILY' CANCER RES. vol. 55, no. 2, 1995, pages 434 - 439, XP001189409
- MIYAUCHI T. ET AL.: 'THE BASIGIN GROUP OF THE IMMUNOGLOBULIN SUPERFAMILY: COMPLETE CONSERVATION OF A SEGMENT IN AND AROUND TRANSMEMBRANE DOMAINS OF HUMAN AND MOUSE BASIGIN AND CHICKEN HT7 ANTIGEN' J. BIOCHEM. vol. 110, no. 5, 1991, pages 770 - 774, XP001056280
- CHEN Z.N. ET AL: 'GENE CLONING OF CD147/HAB18G AND STUDY OF ITS FUNCTION IN HUMAN HEPATOMA CELLS' JOURNAL OF TUMOR MARKER ONCOLOGY vol. 16, no. SUPPL, 01 January 2001, pages 255 - 256, XP008073522
- YANG J. ET AL: 'Preparation and activity of conjugate of monoclonal antibody HAb18 against hepatoma F(ab')(2) fragment and staphylococcal enterotoxin A lian' WORLD JOURNAL OF GASTROENTEROLOGY vol. 7, no. 2, April 2001, pages 216 - 221
- QIU K. ET AL: '99mTc labeled HAb18 McAb Fab fragment for radioimmunoimaging in nude mice bearing human hepatocellular carcinoma' WORLD JOURNAL OF GASTROENTEROLOGY vol. 4, no. 2, 1998, pages 117 - 120

### Sequence listing

<110> CHEN, Zhinan
<120> HAb18G/CD147, ITS ANTAGONIST AND APPLICATION
<130> P2002198C
<150> 01115274.5
   <151> 2001-05-25
<150> 01131735.3
   <151> 2001-09-28
<160> 9
<170> PatentIn version 3.1
<210> 1
   <211> 269
   <212> PRT
   <213> Homo sapiens
<400> 1
   Met Ala Ala Ala Leu Phe Val Leu Leu Gly Phe Ala Leu Leu Gly Thr
   1               5                   10                  15
   His Gly Ala Ser Gly Ala Ala Gly Thr Val Phe Thr Thr Val Glu Asp
               20                  25                  30
   Leu Gly Ser Lys Ile Leu Leu Thr Cys Ser Leu Asn Asp Ser Ala Thr
           35                  40                  45
   Glu Val Thr Gly His Arg Trp Leu Lys Gly Gly Val Val Leu Lys Glu
       50                  55                  60
   Asp Ala Leu Pro Gly Gln Lys Thr Glu Phe Lys Val Asp Ser Asp Asp
   65                  70                  75                  80
   Gln Trp Gly Glu Tyr Ser Cys Val Phe Leu Pro Glu Pro Met Gly Thr
                   85                  90                  95
   Ala Asn Ile Gln Leu His Gly Pro Pro Arg Val Lys Ala Val Lys Ser
               100                 105                 110
   Ser Glu His Ile Asn Glu Gly Glu Thr Ala Met Leu Val Cys Lys Ser
           115                 120                 125
   Glu Ser Val Pro Pro Val Thr Asp Trp Ala Trp Tyr Lys Ile Thr Asp
       130                 135                 140
   Ser Glu Asp Lys Ala Leu Met Asn Gly Ser Glu Ser Arg Phe Phe Val
   145                 150                 155                 160
   Ser Ser Ser Gln Gly Arg Ser Glu Leu His Ile Glu Asn Leu Asn Met
   165 170 175
   Glu Ala Asp Pro Gly Gln Tyr Arg Cys Asn Gly Thr Ser Ser Lys Gly
   180 185 190
   Ser Asp Gln Ala Ile Ile Thr Leu Arg Val Arg Thr His Leu Ala Ala
   195 200 205
   Leu Trp Pro Phe Leu Gly Ile Val Ala Glu Val Leu Val Leu Val Thr
   210 215 220
   Ile Ile Phe Ile Tyr Glu Lys Arg Arg Lys Pro Glu Asp Val Leu Asp
   225 230 235 240
   Asp Asp Asp Ala Gly Ser Ala Pro Leu Lys Ser Ser Gly Gln His Gln
   245 250 255
   Asn Asp Lys Gly Lys Asn Val Arg Gln Arg Asn Ser Ser
   260 265
<210> 2
   <211> 12
   <212> PRT
   <213> Artificial
<220>
<400> 2
   Met Thr His Asp Pro Val Ile Ser Leu Pro Thr Thr
   1 5 10
<210> 3
   <211> 12
   <212> PRT
   <213> Artificial
<220>
<400> 3
   Leu His Arg His Ser His Gly His Ser Tyr Lys Ser
   1 5 10
<210> 4
   <211> 12
   <212> PRT
   <213> Artificial
<220>
<400> 4
   Gly His Trp His Asn His Arg His Gln Ala Pro Leu
   1 5 10
<210> 5
   <211> 12
   <212> PRT
   <213> Artificial
<220>
<400> 5
   Lys Tyr Pro His Gln His Leu His Met His Asp Ser
   1 5 10
<210> 6
   <211> 12
   <212> PRT
   <213> Artificial
<220>
<400> 6
   Ile Gly Trp His Tyr Tyr Leu Arg Thr Gln His Ser
   1 5 10
<210> 7
   <211> 12
   <212> PRT
   <213> Artificial
<220>
<400> 7
   Tyr Pro Phe His His Lys His Trp His Arg Pro Asn
   1 5 10
<210>
   <211> 12
   <212> PRT
   <213> Artificial
<220>
<400> 8
<210> 9
   <211> 12
   <212> PRT
   <213> Artificial
<220>
<400> 9
   Met His Lys His Pro His Gly Ser Gln Gly Pro Thr
   1               5                   10
<210> 10
   <211> 12
   <212> PRT
   <213> Artificial
<220>
<400> 10
   Tyr Lys Leu Pro Gly His His His His Tyr Arg Pro
   1               5                   10

## Description

### FIELD OF THE INVENTION:

The present invention belongs to the field of biotechnology and biomedicine. In particular, the present invention relates to a new protein molecule HAb18G/CD147 identified from hepatocellular carcinoma tissue, the expression plasmid vector of antisense RNA directed to HAb18G/CD147 transcript, and antagonistic peptides for HAb18G/CD147.

### BACKGROUND OF THE INVENTION:

It is estimated that the total incidence of various types of cancer is about 10 million new cases per year, including 1.8 million in China with mortality up to 77.8% (on average, 1.3 people die of cancer in every minute). Moreover, the incidence still tends to increase. It is estimated by WHO that there are about 6 million cases dying of cancer every year, which accounts for 12% of the total number of deaths worldwide. In China, malignant tumor is the second leading cause of death with mortality accounting for 21.85% of the total in 1995. Some cancers have high incidence such as gastric carcinoma (21.76%), hepatocellular carcinoma (17.83%), lung cancer (15.19%), esophageal carcinoma (15.02%) and so on. Tumor's high recurrence and high metastasis has long been a challenge, for which no effective therapy has yet been developed.

At present, one of the focuses on antitumor therapy is to inhibit tumor invasion and metastasis. Tumor invasion and metastasis is a complex process involving multi-phases and multi-factors, including protease; angiogenesis factor, adhesion molecules (such as integrin, selection and E-cadherin); migration factor; signal transduction pathway and related molecules, etc.

During invasion and metastasis, tumor cells induce interstitial cells to secrete proteinase, which degrades extracelluar matrix (ECM). Four major proteinases involved are serine proteinases (such as PAS), cysteine proteinases (such as cathepsin B), aspartic acid proteinases (such as pepsin) and matrix metalloproteinase (MMPs). Among these proteinases, MMPs are of the most importance. Many experimental evidences have shown that the expression and activity of MMPs in malignant tumor tissue are higher than those in normal tissue and show a positive correlation with the degree of the malignancy of tumors. (Davie a B, Miles DW, et al. British cancer, 1993; 67:1126-1131). The MMP family consists of at least 20 enzymes and has the potential to degrade all of the protein and proteoglycan components of the ECM. The roles of MMPs can be summarized as follows:(1) MMPs degrade the components of the ECM and disrupt the barrier of tumor growth. (2) MMPs cleavage basement membrane and promote cancer to cross blood vessel and enter circulation. (3) MMPs promote tumor angiogenesis or the formulation of the metastatic foci by remodeling the ECM.

Since MMPs play a pivotal role in facilitating the metastasis of cancer, the source of MMPs remains the hot spot of research. The findings of many laboratories show that CD147 molecule relates to tumor cell membrane and CD147 stimulates tumor interstitial cells to produce MMPs. CD147 not only stimulates the production of MMPs but also forms a complex with MMPs at the tumor cell surface. Presentation of MMPs complexed to EMMPRIN at the tumor cell surface may be important in modifying the tumor cell pericellular matrix to promote invasion. (Guo H, et al. Cancer Res, 2000; 60(4): 888-891).

At present, proteinase inhibitors were employed to block the degradation of ECM. Natural proteinase inhibitors-tissue inhibitors of metalloproteinases (TIMPs) can bind with MMPs and inhibit the activities of MMPs. However, TIMPs are difficult to be extracted because their contents in tissues are very low. Recombinant TIMPs also have some disadvantages such as prone to degradation as well as malabsorption when administered orally. Synthetic pseudopeptide inhibitors of metalloproteinases, such as Batimastat (BB-94), can inhibit MMPs induced by tumor cells only at early stage and can hardly inhibit the activity of quantities of MMPs at the advanced stage, which results in phase III clinical trial failure.

The inventors have been engaged in the research for hepatocellular carcinoma and immumo-targeted drugs for many years and successfully obtained the specific anti-hepatocellular carcinoma monoclonal antibody HAb18 by immunizing mice with fresh human hepatocellular carcinoma tissue to obtain hybridoma. Using HAb18 as a ligand, hepatocellular carcinoma-associated antigen HAb18G was purified by affinity chromatography. HAb18 was used to screen human hepatocellular carcinoma cDNA expression library and the corresponding coding sequence was obtained (Chen et al., J. Cell. Mol. Immunol., 1999; 15(1):34 and Chen et al., Journal of Tumor Marker Oncology, 2001; 16 (suppl): 255). By searching Genebank, it is found that the amino acid sequence of HAb18G is homologous to that of the leukocyte differentiation antigen CD147 molecule, so the HAb18G molecule belongs to CD147 family. Therefore, the HAb18G molecule was named HAb18G/CD147. Similar molecules were also found in several tissues with different functions such as the surface of the peripheral blood granulocyte of the rheumatoid arthritis and reactive arthritis (Watcharak, Eddafiebiger, Stepanoval. J.Immunology. 1992 (3): 847), lung cancer (Guo H, Zucker S et al. J Biol Chem, 1997; 272(1): 24), brain (Schlosshauer B, Herzog K.H. J Cell Biol. 1990; 110(4): 1261) and so on.

The finding of Matrigel-boyden chamber and gelatin zymography showed that the role of HAb18G/CD147 in hepatocellular carcinoma is to promote hepatocellular carcinoma cells invasion and metastasis, which is identical to the roles of CD147 in other tumors. The mechanism is that HAb18G/CD147 induces hepatocellular carcinoma interstitial cells to produce MMPs, which degrades pericellular matrix components to promote hepatocellular carcinoma cells metastasis. HAb18G/CD147 is abundantly expressed in hepatocellular carcinoma and other tumor tissues while scarcely in normal tissues, so blocking the activity of HAb18G/CD147 may inhibit tumor metastasis.

Rheumatoid arthritis (RA) is characterized by high disable rate, and affects more than 3 million Chinese adults every year with incidence of about 0.3%. In addition, childhood rheumatoid arthritis is the common connective tissue disease in children and its clinical features include hypertrophic and corroding synovitis involving joints of all over the body with recurrent and progressive pathological changes. RA is considered as a "limited malignant tumor" with hypertrophic and destructive pathological changes, and the spontaneous remission is extremely rare. At the advanced stage, the damage of the articular cartilage and bone results in ankylosis, deformity and dysfunction. The pathological changes can also offend the connective tissues, such as serous membrane, heart and lungs, artery, nerve and eyes. So far there is no effective cure for RA. Since the 1990s, researchers have adopted the combined treatment which has shown some advantages in alleviating patients' symptoms and improving their life quality. But the disease is not cured. Therefore, the urgent problem to be solved in the therapy of RA is to find ways to inhibit corrosion to cartilage and bone. The histopathologic characteristic of RA is over-hyperplasia of synovial lining and infiltration of a large number of inflammatory mononuclear cells under the synovial lining. Fibroblasts like synovial cells and synovial tissue macrophage play a very important role in joint damage and both cells can produce a great deal of MMPs. MMPs can degrade all ECM components of synovium, articular cartilage and bone under cartilage with synergy manner. The up-regulation of CD147 expression induces MMP-1, MMP-2 and MMP-3 production and results in the imbalance between MMPs and TIMPs, and in the end causes RA joint damage.

MMPs involves in RA joint damage by directly degrading cartilage, bone tissue and indirectly accelerating angiogenesis. Inhibiting MMPs production is one of the pathway for therapy of RA. In addition, incidence of osteoarthritis is on the rise because it relates to aging. With the elongation of the life span and the incidence rate rising, MMPs produced by chondrocyte, synovial cell and fibroblast also play an important role in osteoarthritis. However, the clinical trial results of anti-ECM degradation of the proteinase inhibitor are disappointing. Therefore, screening effective CD147 antagonistic peptides to inhibit MMPs production is a new road and a new trend for RA and osteoarthritis therapy.

Acquired immunodeficiency syndrome (AIDS) has emerged as one of the most deadly diseases of mankind. Since the epidemic of AIDS, about 60 million people have been infected. AIDS has become the fourth deadly disease in the world and there is no specific therapy for it so far.

Several reports suggested that CD147 is a cell surface receptor for extracellular Cyclophlin A (CyPA). Human immunodeficiency virus (HIV-1) binds with CyPA to form a complex, HIV-1-associated CyPA. And the interaction between HIV-1-associated CyPA and CD147 on target cells significantly enhances the HIV-1 infection. And it is a key step for the prevention and treatment of AIDS to inhibit HIV-1 to enter cells.

HIV is the pathogen for AIDS and CD147 appears to be required for efficient infection by HIV-1. So using effective CD147 antagonistic peptides to prevent HIV from binding with CD147 may provide a new means for the prevention and therapy of AIDS.

Several studies showed that CD147 was involved in arteriosclerosis and dilated cardiomyopathy. Therefore, CD147 antagonistic peptides also have potential application for the prevention and treatment of arteriosclerosis and dilated cardiomyopathy.

### SUMMARY OF THE INVENTION:

The present invention provides an antagonistic peptide for the HAb18G/CD147. The antagonistic peptide comprises an amino acid sequence selected from:
(1) Met Thr His Asp Pro Val Ile Ser Leu Pro Thr Thr (SEQ ID NO:2);
(2) Leu His Arg His Ser His Gly His Ser Tyr Lys Ser (SEQ ID NO:3);
(3) Gly His Trp His Asn His Arg His Gln Ala Pro Leu (SEQ ID NO:4);
(4) Lys Tyr Pro His Gln His Leu His Met His Asp Ser (SEQ ID NO:5);
(5) Ile Gly Trp His Tyr Tyr Leu Arg Thr Gln His Ser (SEQ ID NO:6);
(6) Tyr Pro Phe His His Lys His Trp His Arg Pro Asn (SEQ ID NO:7);
(7) Ala Asn Ile Val Pro Ile His Ala Asn His Phe Gln (SEQ ID NO:8);
(8) Met His Lys His Pro His Gly Ser Gln Gly Pro Thr (SEQ ID NO:9); and
(9) Tyr Lys Leu Pro Gly His His His His Tyr Arg Pro (SEQ ID NO:10).

Further disclosed herein are a protein molecule HAb18G/CD147 identified from hepatocellular carcinoma tissue comprising the amino acid sequence as set forth in SEQ ID NO:1, and an antisense vector PCI-as HAb18G directed to the HAb18G/CD147 transcript.

In another aspect, the present invention provides a method for preventing and treating the recurrence and metastasis of cancer, for preventing and treating RA and osteoarthritis, for preventing and treating HIV infection and AIDS, or for preventing and treating arteriosclerosis and dilated cardiomyopathy in a subject, by administering to the subject an effective dose of the antagonistic peptide for HAb18G/CD147 of the invention.

And in a further aspect, the present invention provides a pharmaceutical composition for preventing and treating the recurrence and metastasis of cancer, for preventing and treating RA and osteoarthritis, for preventing and treating HIV infection and AIDS, or for preventing and treating arteriosclerosis and dilated cardiomyopathy in a subject, which comprises an effective dose of the antagonistic peptide of the invention and a pharmaceutically acceptable carrier.

### BRIEF DESCRIPTION OF THE DRAWINGS:

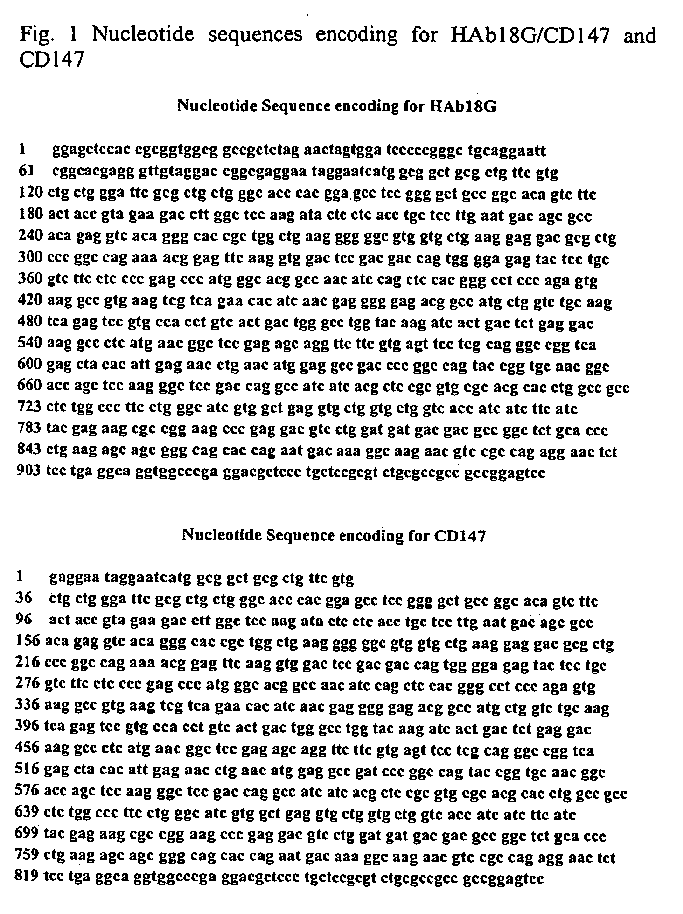

Figure 1: Comparison of the nucleotide sequences encoding for HAb18G/CD147 and CD147.

### DETAILED DESCRIPTION OF THE INVENTION:

The present invention is described in detail as follows.

Disclosed herein is a new protein molecule HAb18G/CD147 identified from hepatocellular carcinoma tissue.

### 1. Purification of natural antigen HAb18G/CD147

(1) A monoclonal antibody HAb18 was bound to Sepharose-4B column;
(2) HHCC cells were disrupted, centrifuged and dialyzed to obtain a crude sample of membrane antigens. The obtained crude sample was then loaded onto the Sepharose-4B column prepared in (1), and after the steps of binding, eluting and collecting, purified HAb18G/CD147 antigen was obtained in the end.

The HAb18G/CD147 antigen appeared to be a single band with a molecular weight of 60 kD as judged by SDS-PAGE and Western blotting.

### 2. Gene cloning of HAb18G/CD147

HAb18G is a newly identified hepatocellular carcinoma-associated antigen. The gene encoding this antigen is cloned by immunoscreening the hepatocellular carcinoma cDNA expression library using the hepatocellular carcinoma monoclonal antibody HAb18. The amino acid sequence of HAb18G/CD147 is composed of 269 amino acids and contains, from N-terminal to C-terminal, a signal peptide sequence rich in hydrophobic residues, an extracellular functional domain, a transmembrane domain consisting of the 24 amino acid residues from site 206 to site 229, and an intracellular domain consisting of 39 amino acid residues at the C-terminal. The HAb18G/CD147 contains a large number of posttranslational modifications including glycosylation, the mode of which varies with species and tissues. Therefore, although the primary structure of the HAb18G/CD147 identified from hepatocellular carcinoma tissue is homologous to that of CD147, their glycosylation modes may be quite different, resulting in the difference in function between HAb18G/CD147 and the similar molecules derived from other tissues.

### 3. The functions of HAb18G/CD147

By applying the purified HAb18G/CD147 to human fibroblasts, the inventors found that the HAb18G/CD147 is capable of stimulating human fibroblasts to produce MMPs. The experiments were carried out as follows:

Purified HAb18G/CD147 was added to the cultured human fibroblasts and the supernatant of the culture was collected and then subjected to SDS-PAG electrophoresis in a separation gel containing 1mg/ml gelatin. Different from the conventional SDS-PAGE, no DTT was added to the sample and furthermore, the sample was not boiled. After electrophoresis, the gel was washed twice (45 min/each time) at room temperature and washed twice (20 min/each time) with 0.1mol/L NaCl, and then incubated for 24 hours in an incubation solution at 37°C. The gel was stained with 0.5% (w/v) Coomassie Blue and then destained. MMPs were detected as clear bands against a blue background. The finding of gelatin zymography showed that after being stimulated by purified HAb18G/CD147, the human fibroblasts secreted three different MMPs, namely MMP-9 (pro-MMP-9 with molecular weight of 92 KD, active MMP-9 of 86 KD), MMP-2 (pro-MMP-2 with molecular weight of 72 KD, active MMP-2 of 68 KD) and MT1-MMP with molecular weight of 43 KD.

It can be seen from the above experiments that HAb18G/CD147 stimulates human fibroblasts to produce several kinds of MMPs and the role of MMPs is to enhance the invasion and metastasis ability of hepatocellular carcinoma cells. The function of HAb18G/CD147 suggests that reducing the expression of HAb18G/CD147 may provide a new way for the gene therapy of hepatocellular carcinoma, for example, by means of antisense.

Also disclosed herein is an antisense vector PCI-as HAb18G/CD147 directed to the HAb18G/CD147 transcript.

### 1. Construction of antisense plasmids vector to HAb18G/CD147 transcript

Full length cDNA of HAb18G/CD147 prepared by digesting pBluescript ks (+/-)/HAb18G (homemade) with Xbal and Xhol was inserted reversely into eukaryotic expressing vector PCI-neo (Promega company, Madison, WI, USA) digested with the same restriction endonucleases. Because of reverse order of enzyme-cutting, HAb18G/CD147 cDNA was linked to PCI-neo vector with the reverse order. The antisense RNA complementary to HAb18G/CD147 mRNA was transcripted. This resulted in the blockage of the translation of HAb18G/CD147 mRNA and therefore, the expression of HAb18G/CD147 was inhibited.

### 2. Functions of antisense plasmids vector to HAb18G/CD147 transcript

### a. Comparison of HAb18G/CD147 expression in transfected and non-transfected hepatocellular carcinoma cells.

The expression of HAb18G/CD147 on hepatocellular carcinoma cells transfected with PCI-asHAb18G decreased as demonstrated by immunohistochemical staining and FACS analysis. It was found that the antisense RNA blocked the expression of HAb18G/CD147 with a blocking rate as high as 95%.

### b. Inhibition of MMPs

HHCCs blocked by antisense RNA were co-cultured with fibroblasts. The supernatant was collected and assayed by SDS-PAGE Gelatin Zymography. It was found that the production of MMPs in HHCCs decreased significantly by the antisense RNA.

### c. Reconstituted basement membrane invasion assay

Matrigel (main component was type IV collagen, BD Company) was added onto the inner surface of Boyden Chambers (Millipore) to form the reconstituted basement membrane. Cells (HHCC cells and human fibroblasts) and antisense RNA were added and the chambers were put in the 24-well plates and co-cultured overnight. Cells infiltrated through the reconstituted basement membrane and appeared on the outer surfaces of the membrane were stained and counted under high-power microscope. The results showed that the antisense RNA targeting HAb18G/CD147 mRNA interfered the translation and expression of HAb18G/CD147, weakened the productions of MMPs, and inhibited the invasion of hepatocellular carcinoma cells through reconstituted basement membrane.

The findings mentioned above indicated that antisense plasmids vector to HAb18G/CD147 blocked the expression of HAb18G/CD147, Inhibited hepatocellular carcinoma cells invasion and changed the biologic behaviors of these tumor cells. Thus the antisense plasmids vector of HAb18G/CD147 (PCI-as HAb18G/CD147) is shown to be capable of effectively blocking HAb18G/CD147 expression and inhibiting hepatocellular carcinoma invasion and metastasis.

The present invention provides antagonistic peptides for HAb18G/CD147 which were obtained by methods as follows:
1. Purification of HAb18G/CD147
   (1) HAb18 monoclonal antibody was bound to Sepharose-4B.
   (2) A large number of HHCC cells were disrupted, centrifuged and dialyzed to prepare crude membrane antigen and the crude membrane antigen was loaded onto the affinity chromatography column of step (1). After the steps of binding, eluting and collecting, pure HAb18G/CD147 was obtained in the end.
   SDS-PAGE and Western blot analysis with HAb18 detected a single band with a molecular weight of 60 kD.
2. Hepatocellular carcinoma monoclonal antibody HAb18 was used to screen hepatocellular carcinoma cDNA library and the corresponding coding sequence of HAb18G/CD147 was obtained. It has been identified that the sequence of HAb18G is highly homologous to that of CD147.
3. Nine binding peptides of HAb18G/CD147 were obtained by screening a 12mer phage display library with purified HAb18G/CD147 and their sequences are as follows:
   (1) Met Thr His Asp Pro Val Ile Ser Leu Pro Thr Thr (SEQ ID NO:2);
   (2) Leu His Arg His Ser His Gly His Ser Tyr Lys Ser (SEQ ID NO:3);
   (3) Gly His Trp His Asn His Arg His Gln Ala Pro Leu (SEQ ID NO:4);
   (4) Lys Tyr Pro His Gln His Leu His Met His Asp Ser (SEQ ID NO:5);
   (5) Ile Gly Trp His Tyr Tyr Leu Arg Thr Gln His Ser (SEQ ID NO:6);
   (6) Tyr Pro Phe His His Lys His Trp His Arg Pro Asn (SEQ ID NO:7);
   (7) Ala Asn Ile Val Pro Ile His Ala Asn His Phe Gln (SEQ ID NO:8);
   (8) Met His Lys His Pro His Gly Ser Gin Gly Pro Thr (SEQ ID NO:9); and
   (9) Tyr Lys Leu Pro Gly His His His His Tyr Arg Pro (SEQ ID NO:10);
4. The binding peptides were sequenced and synthesized (Meilian Company in Xi'an) and the purity of these peptides was over 99% by FPLC and MS assay.

The amino acids of the antagonistic peptides mentioned above are L-amino acids. Based on the analysis of three-dimensional structure-activity relationship of antagonistic peptides, the inventors modified HAb18G/CD147 by substitution of L-amino acids with D-amino acids.

### Studies on the activities of antagonistic peptides for HAb18G/CD147

1. *in vitro* studies: reconstituted basement membrane invasion was employed to test the effects of the antagonistic peptides for HAb18G/CD147 on the invasive ability of HHCCs. Antagonistic peptides were co-cultured with HHCCs and fibroblasts on the reconstituted basement membrane for 20 hours. Cells infiltrated through the reconstituted basement membrane were counted under high-power microscope and the inhibitory rate of the antagonistic peptides was calculated. The results are as follows:

| Groups | infiltrative cells | inhibitory rate (%) |
|---|---|---|
| Control | 20.2±18.5 | |
| AP-1 | 4.4±1.5 | 78.2^{a} |
| AP-2 | 28±6.9 | -38.61 |
| AP-3 | 2.0±0.0 | 90.1 |
| AP-4 | 12.2±5.6 | 39.6 |
| AP-5 | 23.2±4.7 | -14.85 |
| AP-6 | 7.6±4.9 | 62.38^{a} |
| AP-7 | 8.8±4.7 | 56.44^{a} |
| AP-8 | 6.4±2.2 | 68.32^{a} |
| AP-9 | 3.8±2.1 | 81.19^{a} |

| | | |
|---|---|---|
| ^{a}*P*<0.05 *v*.*s*. Control | | |

Six (AP-1, 3, 6, 7, 8, 9) of these nine antagonistic peptides significantly inhibited hepatocellular carcinoma cells invasion and metastasis with the highest inhibitory rate up to 90.1 %. Gelatin zymography was also employed to test the inhibitory effects of antagonistic peptides on MMPs production and the results of Gelatin Zymography were the same as that of the reconstituted basement membrane invasion experiment. The results of anti-adhesion assay showed that HAb18G/CD147 antagonistic peptides inhibited tumor cells adhesion to ECM components (laminin, fibronectin, Collagen IV) with different degrees.
The *in vitro* results showed that HAb18G/CD147 stimulated fibroblasts to produce MMPs and enhanced the activity and amount of MMPs in tumor tissue, leading to over degradation of extracellular matrix and collagen protein and thus allowing tumor cells to cross the basement membrane, invade and spread in the surrounding tissues. The results of the anti-invasion experiment showed that the inhibitory rate of antagonistic peptides was from -39.0% to 90.1%. Moreover, the findings of Gelatin Zymography showed that antagonistic peptides inhibited MMPs production.
2. *in vivo* studies: The mice were inoculated with 200 µl HHCC cells (1 X 10⁶/ml) and were then treated with HAb18G/CD147 antagonistic peptides (i.v.) at the dose of 2.5mg/kg weight. The results showed that AP-1 and AP-6 had significant inhibitory effects on tumorigenesis of hepatocellular carcinoma cells. It is observed that on the 11th day from inoculation, tumors formed in all mice of control group while no tumor formed in the mice of therapeutic group treated wtih AP-6. Moreover, the average survival time of mice in therapeutic group was two times as long as that of the control group. A high metastatic human hepatocellular carcinoma cell line MHCC97-H was transplanted into the liver of nude mice to investigate the anti-metastasis effects of APs. The results showed that HAb18G/CD147 antagonistic peptides inhibited the spreading of hepatocellular carcinoma cells to the liver, the lung and abdominal cavity. The inhibitory effects of HAb18G/CD147 antagonistic peptides on angiogenesis in chick embryo chorioallantoic membrane (CAM) as well as on angiogenesis induced by Matrigel plug were detected. The results showed that HAb18G/CD147 antagonistic peptides significantly blocked the angiogenesis in CAM and that induced by matrigel plug in C57/BL mice.
3. HAb18G/CD147 expression in synovium: HAb18G/CD147 expression in RA and osteoarthritis synovium tissue was detected by immunohistochemistry and the results showed that HAb18G/CD147 was over-expressed in RA synovium cells. Flowcytometry analysis using Triple immunofluorescence labelling demonstrated that HAb18G/CD147 was highly expressed in samples from RA patients including peripheral blood cells, monocyte/macrophage cells in synovial fluid and activated T cells. Moreover, CCR5 and CXCR3 ligands were highly expressed in the joint cavity of RA patients. CCR5 and CXCR3 ligands are capable of inducing CCR5 and CXCR3 expressing Th1 subpopulation and monocyte/macrophage cells to selectively aggregate in joint cavities and subsequently participate in joint damage in RA. The finding of gelatin zymography and reconstituted basement membrane invasion showed that, by producing a large number of HAb18G/CD147, monocyte/macrophage cells interacted with fibroblasts, thereby the joint damage was aggravated. Therefore, the antagonistic peptides for HAb18G/CD147 are expected to have potential therapeutic value in the treatment of RA and osteoarthritis.

Based on the above studies, effective dose of PCI-as HAb18G/CD147 or antagonistic peptides for HAb18G/CD147 or the combination thereof may be used for preventing and treating the recurrence and metastasis of cancers, RA and osteoarthritis, HIV infection and AIDS, as well as arteriosclerosis and dilated cardiomyopathy.

In addition, the invention also provides a pharmaceutical composition for preventing and treating of recurrence and metastasis of cancer, RA and osteoarthritis, HIV infection and AIDS, as well as arteriosclerosis and dilated cardiomyopathy. The pharmaceutical composition of the invention includes antagonistic peptides for HAb18G/CD147 as defined above and a pharmaceutically acceptable vector. The pharmaceutical composition can be produced with general methods known in this field. Furthermore, the inventors found that the half-lives, activity and *in vivo* bioavailability of the antagonistic peptides for HAb18G/CD147 can be increased by modification with nanometer liposome

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. An antagonistic peptide for the protein HAb18G, wherein said antagonistic peptide comprises an amino acid sequence selected from:
Met Thr His Asp Pro Val Ile Ser Leu Pro Thr Thr (SEQ ID NO:2);
Gly His Trp His Asn His Arg His Gln Ala Pro Leu (SEQ ID NO:4);
Lys Tyr Pro His Gln His Leu His Met His Asp Ser (SEQ ID NO:5);
Tyr Pro Phe His His Lys His Trp His Arg Pro Asn (SEQ ID NO:7);
Ala Asn Ile Val Pro Ile His Ala Asn His Phe Gln (SEQ ID NO:8);
Met His Lys His Pro His Gly Ser Gln Gly Pro Thr (SEQ ID NO:9); and
Tyr Lys Leu Pro Gly His His His His TyrArg Pro (SEQ ID NO:10).

2. The antagonistic peptide according to claim 1, wherein the antagonistic peptide is synthesized by solid phase synthesis.

3. The antagonistic peptide according to claim 1, wherein the amino acids of said antagonistic peptide are L-amino acids.

4. The antagonistic peptide according to claim 1, wherein the amino acids of said antagonistic peptide are D-amino acids.

5. Use of an antagonistic peptide of any one of claims 1 to 4 in preparation of a pharmaceutical composition for preventing and treating the recurrence and metastasis of cancer, for preventing and treating RA and osteoarthritis, for preventing and treating HIV infection and AIDS, or for preventing and treating arteriosclerosis and dilated cardiomyopathy.

6. A pharmaceutical composition for preventing and treating the recurrence and metastasis of cancer, for preventing and treating RA and osteoarthritis, for preventing and treating HIV infection and AIDS, or for preventing and treating arteriosclerosis and dilated cardiomyopathy in a subject in need of such treatment, comprising an effective dose of the antagonistic peptide of any one of claims 1 to 4, and a pharmaceutically acceptable carrier.

7. The pharmaceutical composition according to claim 6, comprising the antagonistic peptide according to any one of claims 1 to 4 entrapped within nanometer liposomes.

## Patentansprüche

1. Antagonistisches Peptid für das Protein HAb18G, wobei das antagonistische Peptid eine Aminosäuresequenz umfasst, die ausgewählt ist aus:
Met Thr His Asp Pro Val Ile Ser Leu Pro Thr Thr (SEQ ID NO:2);
Gly His Trp His Asn His Arg His Gin Ala Pro Leu (SEQ ID NO:4);
Lys Tyr Pro His Gln His Leu His Met His Asp Ser (SEQ ID NO:5);
Tyr Pro Phe His His Lys His Trp His Arg Pro Asn (SEQ ID NO:7);
Ala Asn Ile Val Pro Ile His Ala Asn His Phe Gln (SEQ ID NO:8);
Met His Lys His Pro His Gly Ser Gln Gly Pro Thr (SEQ ID NO: 9); und
Tyr Lys Leu Pro Gly His His His His Tyr Arg Pro (SEQ ID NO:10).

2. Antagonistisches Peptid nach Anspruch 1, wobei das antagonistische Peptid mittels Festphasensynthese synthetisiert ist.

3. Antagonistisches Peptid nach Anspruch 1, wobei die Aminosäuren des antagonistischen Peptids L-Aminosäuren sind.

4. Antagonistisches Peptid nach Anspruch 1, wobei die Aminosäuren des antagonistischen Peptids D-Aminosäuren sind.

5. Verwendung eines antagonistischen Peptids nach einem der Ansprüche 1 bis 4 in der Herstellung einer pharmazeutischen Zusammensetzung zum Vorbeugen und Behandeln des Wiederauftretens und der Metastase von Krebs, zum Vorbeugen und Behandeln von RA und Osteoarthritis, zum Vorbeugen und Behandeln von HIV-Infektion und AIDS, oder zum Vorbeugen und Behandeln von Arteriosklerose und dilatierter Cardiomyopathie.

6. Pharmazeutische Zusammensetzung zum Vorbeugen und Behandeln des Wiederauftretens und der Metastase von Krebs, zum Vorbeugen und Behandeln von RA und Osteoarthritis, zum Vorbeugen und Behandeln von HIV-Infektion und AIDS, oder zum Vorbeugen und Behandeln von Arteriosklerose und dilatierter Cardiomyopathie in einem Subjekt, das solcher Behandlung bedarf, umfassend eine wirksame Dosis des antagonistischen Peptids nach einem der Ansprüche 1 bis 4, und einen pharmazeutisch verträglichen Träger.

7. Pharmazeutische Zusammensetzung nach Anspruch 6, umfassend das antagonistische Peptid nach einem der Ansprüche 1 bis 4, eingeschlossen in Nanometer-Liposomen.

## Revendications

1. Peptide antagoniste pour la protéine HAb18G, dans lequel ledit peptide antagoniste comprend une séquence d'acides aminés choisie parmi :
Met Thr His Asp Pro Val Ile Ser Leu Pro Thr Thr (SEQ ID NO :2) ;
Gly His Trp His Asn His Arg His Gln Ala Pro Leu (SEQ ID NO :4) ;
Lys Tyr Pro His Gln His Leu His Met His Asp Ser (SEQ ID NO :5) ;
Tyr Pro Phe His His Lys His Trp His Arg Pro Asn (SEQ ID NO :7) ;
Ala Asn Ile Val Pro Ile His Ala Asn His Phe Gln (SEQ ID NO :8) ;
Met His Lys His Pro His Gly Ser Gln Gly Pro Thr (SEQ ID NO :9) ; et
Tyr Lys Leu Pro Gly His His His His Tyr Arg Pro (SEQ ID NO :10).

2. Peptide antagoniste selon la revendication 1, dans lequel le peptide antagoniste est synthétisé par une synthèse en phase solide.

3. Peptide antagoniste selon la revendication 1, dans lequel les acides aminés dudit peptide antagoniste sont des L-acides aminés.

4. Peptide antagoniste selon la revendication 1, dans lequel les acides aminés dudit peptide antagoniste sont des D-acides aminés.

5. Utilisation d'un peptide antagoniste selon l'une quelconque des revendications 1 à 4 dans la préparation d'une composition pharmaceutique pour prévenir et traiter la récidive et les métastase d'un cancer, pour prévenir et traiter la polyarthrite rhumatoïde et l'arthrose, pour prévenir et traiter une infection par le VIH et le SIDA, ou pour prévenir et traiter l'artériosclérose et une cardiomyopathie dilatée.

6. Une composition pharmaceutique pour prévenir et traiter la récidive et les métastase d'un cancer, pour prévenir et traiter la polyarthrite rhumatoïde et l'arthrose, pour prévenir et traiter une infection par le VIH et le SIDA, ou pour prévenir et traiter l'artériosclérose et une cardiomyopathie dilatée chez un sujet ayant besoin d'un tel traitement, comprenant une dose efficace du peptide antagoniste selon l'une quelconque des revendications 1 à 4, et un support pharmaceutiquement acceptable.

7. Composition pharmaceutique selon la revendication 6, comprenant le peptide antagoniste selon l'une quelconque des revendications 1 à 4 piégé à l'intérieur de liposomes nanométriques.
